# DEMANDE DE BREVET EUROPEEN

(11) **EP 2 208 793 A1**
(43) Date de publication de la demande: **21.07.2010**
(21) Numéro de dépôt: 08005332.5
(22) Date de dépôt: 17.12.1993
(51) Int. Cl.: C12Q 1/18, C07K 14/315, C12N 9/00

(54) **Protéine conférant une résistance de type inductible, a des glycopeptides, notamment chez des bactéries à grampositif**

(30) Priorité: 18.12.1992 FR 9215671; 07.07.1993 FR 9308356
(62) Demande divisionnaire de: 94902830.2
(71) Demandeur: Institut Pasteur, 75724 Paris Cédex 15 (FR)
(72) Inventeur: Arthur, Michel, 94110 Arcueil (FR); Dutka-Malen, Sylvie, 94260 Fresnes (FR); Evers, Stefan, 75013 Paris (FR); Courvalin, Patrice, 75015 Paris (FR)
(74) Mandataire: Desaix, Anne

(57) **Abrégé**

L'invention concerne une protéine VanB impliquée chez des bactéries à Gram-positif, dans une résistance à des glycopeptides, en particulier à la vancomycine, cette résistance étant de type inductible par la vancomycine et non inductible par la teicoplanine. L'invention vise aussi l'utilisation de fragments de nucléotides du gène Van B pour la détection de résistances à des glycopeptides.

SEQUENCE LISTING

<110>  INSTITUT PASTEUR

<120>  PROTEIN CONFERRING AN INDUCIBLE RESISTANCE TO GLYCOPEPTIDES,
       PARTICULARLY IN GRAM-POSITIVE BACTERIA

<130>  B01987AAAQ - AD/LV/KN

<140>  EP 08005332.5
<141>  1993-12-17

<150>  FR 9215671
<151>  1992-12-18

<150>  FR 9308356
<151>  1993-07-07

<160>  10

<170>  PatentIn version 3.3

<210>  1
<211>  1140
<212>  DNA
<213>  Enterococcus faecalis / gène Van B


<220>
<221>  CDS
<222>  (68)..(1096)

<400>  1
gagcgtgtgc tgcgagatac cacagaaaac aatcagaatt gtcttaactt tgaaaggagt      60

ttacagc atg aat aaa ata aaa gtc gca att atc ttc ggc ggt tgc tcg      109
        Met Asn Lys Ile Lys Val Ala Ile Ile Phe Gly Gly Cys Ser
        1               5                   10

gag gaa cat gat gtg tcg gta aaa tcc gca ata gaa att gct gcg aac      157
Glu Glu His Asp Val Ser Val Lys Ser Ala Ile Glu Ile Ala Ala Asn
15                  20                  25                  30

att aat act gaa aaa ttc gat ccg cac tac atc gga att aca aaa aac      205
Ile Asn Thr Glu Lys Phe Asp Pro His Tyr Ile Gly Ile Thr Lys Asn
                35                  40                  45

ggc gta tgg aag cta tgc aag aag cca tgt acg gaa tgg gaa gcc gat      253
Gly Val Trp Lys Leu Cys Lys Lys Pro Cys Thr Glu Trp Glu Ala Asp
            50                  55                  60

agt ctc ccc gcc ata ttc tcc ccg gat agg aaa acg cat ggt ctg ctt      301
Ser Leu Pro Ala Ile Phe Ser Pro Asp Arg Lys Thr His Gly Leu Leu
        65                  70                  75

gtc atg aaa gaa aga gaa tac gaa act cgg cgt att gac gtg gct ttc      349
Val Met Lys Glu Arg Glu Tyr Glu Thr Arg Arg Ile Asp Val Ala Phe
    80                  85                  90

ccg gtt ttg cat ggc aaa tgc ggg gag gat ggt gcg ata cag ggt ctg      397
Pro Val Leu His Gly Lys Cys Gly Glu Asp Gly Ala Ile Gln Gly Leu
95                  100                 105                 110

ttt gaa ttg tct ggt atc ccc tat gta ggc tgc gat att caa agc tcc      445
Phe Glu Leu Ser Gly Ile Pro Tyr Val Gly Cys Asp Ile Gln Ser Ser
                115                 120                 125

gca gct tgc atg gac aaa tca ctg gcc tac att ctt aca aaa aat gcg      493

Ala Ala Cys Met Asp Lys Ser Leu Ala Tyr Ile Leu Thr Lys Asn Ala
            130                 135                 140

ggc atc gcc gtc ccc gaa ttt caa atg att gaa aaa ggt gac aaa ccg      541
Gly Ile Ala Val Pro Glu Phe Gln Met Ile Glu Lys Gly Asp Lys Pro
        145                 150                 155

gag gcg agg acg ctt acc tac cct gtc ttt gtg aag ccg gca cgg tca      589
Glu Ala Arg Thr Leu Thr Tyr Pro Val Phe Val Lys Pro Ala Arg Ser
    160                 165                 170

ggt tcg tcc ttt ggc gta acc aaa gta aac agt acg gaa gaa cta aac      637
Gly Ser Ser Phe Gly Val Thr Lys Val Asn Ser Thr Glu Glu Leu Asn
175                 180                 185                 190

gct gcg ata gaa gca gca gga caa tat gat gga aaa atc tta att gag      685
Ala Ala Ile Glu Ala Ala Gly Gln Tyr Asp Gly Lys Ile Leu Ile Glu
                195                 200                 205

caa gcg att tcg ggc tgt gag gtc ggc tgc gcg gtc atg gga aac gag      733
Gln Ala Ile Ser Gly Cys Glu Val Gly Cys Ala Val Met Gly Asn Glu
            210                 215                 220

gat gat ttg att gtc ggc gaa gtg gat caa atc cgg ttg agc cac ggt      781
Asp Asp Leu Ile Val Gly Glu Val Asp Gln Ile Arg Leu Ser His Gly
        225                 230                 235

atc ttc cgc atc cat cag gaa aac gag ccg gaa aaa ggc tca gag aat      829
Ile Phe Arg Ile His Gln Glu Asn Glu Pro Glu Lys Gly Ser Glu Asn
    240                 245                 250

gcg atg att atc gtt cca gca gac att ccg gtc gag gaa cga aat cgg      877
Ala Met Ile Ile Val Pro Ala Asp Ile Pro Val Glu Glu Arg Asn Arg
255                 260                 265                 270

gtg caa gaa acg gca aag aaa gta tat cgg gtg ctt gga tgc aga ggg      925
Val Gln Glu Thr Ala Lys Lys Val Tyr Arg Val Leu Gly Cys Arg Gly
                275                 280                 285

ctt gct cgt gtt gat ctt ttt ttg cag gag gat ggc ggc atc gtt cta      973
Leu Ala Arg Val Asp Leu Phe Leu Gln Glu Asp Gly Gly Ile Val Leu
            290                 295                 300

aac gag gtc aat acc ctg ccc ggt ttt aca tcg tac agc cgc tat cca      1021
Asn Glu Val Asn Thr Leu Pro Gly Phe Thr Ser Tyr Ser Arg Tyr Pro
        305                 310                 315

cgc atg gcg gct gcc gca gga atc acg ctt ccc gca cta att gac agc      1069
Arg Met Ala Ala Ala Ala Gly Ile Thr Leu Pro Ala Leu Ile Asp Ser
    320                 325                 330

ctg att aca ttg gcg ata gag agg tga cccgtatgga aaatggtttt      1116
Leu Ile Thr Leu Ala Ile Glu Arg
335                 340

ttgtttttta gatgaaatgt tgca      1140

<210>  2
<211>  342
<212>  PRT
<213>  Enterococcus faecalis / gène Van B

<400>  2

Met Asn Lys Ile Lys Val Ala Ile Ile Phe Gly Gly Cys Ser Glu Glu
1               5                   10                  15

His Asp Val Ser Val Lys Ser Ala Ile Glu Ile Ala Ala Asn Ile Asn
            20                  25                  30

Thr Glu Lys Phe Asp Pro His Tyr Ile Gly Ile Thr Lys Asn Gly Val
        35                  40                  45

Trp Lys Leu Cys Lys Lys Pro Cys Thr Glu Trp Glu Ala Asp Ser Leu
    50                  55                  60

Pro Ala Ile Phe Ser Pro Asp Arg Lys Thr His Gly Leu Leu Val Met
65                  70                  75                  80

Lys Glu Arg Glu Tyr Glu Thr Arg Arg Ile Asp Val Ala Phe Pro Val
                85                  90                  95

Leu His Gly Lys Cys Gly Glu Asp Gly Ala Ile Gln Gly Leu Phe Glu
            100                 105                 110

Leu Ser Gly Ile Pro Tyr Val Gly Cys Asp Ile Gln Ser Ser Ala Ala
        115                 120                 125

Cys Met Asp Lys Ser Leu Ala Tyr Ile Leu Thr Lys Asn Ala Gly Ile
    130                 135                 140

Ala Val Pro Glu Phe Gln Met Ile Glu Lys Gly Asp Lys Pro Glu Ala
145                 150                 155                 160

Arg Thr Leu Thr Tyr Pro Val Phe Val Lys Pro Ala Arg Ser Gly Ser
                165                 170                 175

Ser Phe Gly Val Thr Lys Val Asn Ser Thr Glu Glu Leu Asn Ala Ala
            180                 185                 190

Ile Glu Ala Ala Gly Gln Tyr Asp Gly Lys Ile Leu Ile Glu Gln Ala
        195                 200                 205

Ile Ser Gly Cys Glu Val Gly Cys Ala Val Met Gly Asn Glu Asp Asp
    210                 215                 220

Leu Ile Val Gly Glu Val Asp Gln Ile Arg Leu Ser His Gly Ile Phe
225                 230                 235                 240

Arg Ile His Gln Glu Asn Glu Pro Glu Lys Gly Ser Glu Asn Ala Met
                245                 250                 255

Ile Ile Val Pro Ala Asp Ile Pro Val Glu Glu Arg Asn Arg Val Gln
            260                 265                 270

Glu Thr Ala Lys Lys Val Tyr Arg Val Leu Gly Cys Arg Gly Leu Ala
        275                 280                 285

Arg Val Asp Leu Phe Leu Gln Glu Asp Gly Gly Ile Val Leu Asn Glu
    290                 295                 300

Val Asn Thr Leu Pro Gly Phe Thr Ser Tyr Ser Arg Tyr Pro Arg Met
305                 310                 315                 320

Ala Ala Ala Ala Gly Ile Thr Leu Pro Ala Leu Ile Asp Ser Leu Ile
                325                 330                 335

Thr Leu Ala Ile Glu Arg
            340

<210>  3
<211>  196
<212>  PRT
<213>  Artificial

<220>
<223>  Van B fragment

<400>  3

Leu Phe Glu Leu Ser Gly Ile Pro Tyr Val Gly Cys Asp Ile Gln Ser
1               5                   10                  15

Ser Ala Ala Cys Met Asp Lys Ser Leu Ala Tyr Ile Leu Thr Lys Asn
            20                  25                  30

Ala Gly Ile Ala Val Pro Glu Phe Gln Met Ile Glu Lys Gly Asp Lys
        35                  40                  45

Pro Glu Ala Arg Thr Leu Thr Tyr Pro Val Phe Val Lys Pro Ala Arg
    50                  55                  60

Ser Gly Ser Ser Phe Gly Val Thr Lys Val Asn Ser Thr Glu Glu Leu
65                  70                  75                  80

Asn Ala Ala Ile Glu Ala Ala Gly Gln Tyr Asp Gly Lys Ile Leu Ile
                85                  90                  95

Glu Gln Ala Ile Ser Gly Cys Glu Val Gly Cys Ala Val Met Gly Asn
            100                 105                 110

Glu Asp Asp Leu Ile Val Gly Glu Val Asp Gln Ile Arg Leu Ser His
        115                 120                 125

Gly Ile Phe Arg Ile His Gln Glu Asn Glu Pro Glu Lys Gly Ser Glu
    130                 135                 140

Asn Ala Met Ile Ile Val Pro Ala Asp Ile Pro Val Glu Glu Arg Asn
145                 150                 155                 160

Arg Val Gln Glu Thr Ala Lys Lys Val Tyr Arg Val Leu Gly Cys Arg

165                 170                 175

Gly Leu Ala Arg Val Asp Leu Phe Leu Gln Glu Asp Gly Gly Ile Val
            180                 185                 190

Leu Asn Glu Val
        195

<210>  4
<211>  589
<212>  DNA
<213>  Artificial

<220>
<223>  Van B fragment

<400>  4
tctgtttgaa ttgtctggta tcccctatgt aggctgcgat attcaaagct ccgcagcttg      60

catggacaaa tcactggcct acattcttac aaaaaatgcg ggcatcgccg tccccgaatt     120

tcaaatgatt gaaaaaggtg acaaaccgga ggcgaggacg cttacctacc ctgtctttgt     180

gaagccggca cggtcaggtt cgtcctttgg cgtaaccaaa gtaaacagta cggaagaact     240

aaacgctgcg atagaagcag caggacaata tgatggaaaa atcttaattg agcaagcgat     300

ttcgggctgt gaggtcggct gcgcggtcat gggaaacgag gatgatttga ttgtcggcga     360

agtggatcaa atccggttga gccacggtat cttccgcatc catcaggaaa acgagccgga     420

aaaaggctca gagaatgcga tgattatcgt tccagcagac attccggtcg aggaacgaaa     480

tcgggtgcaa gaaacggcaa agaaagtata tcgggtgctt ggatgcagag ggcttgctcg     540

tgttgatctt tttttgcagg aggatggcgg catcgttcta aacgaggtc                 589

<210>  5
<211>  17
<212>  DNA
<213>  Artificial

<220>
<223>  Amorce 1

<400>  5
atgggaagcc gatagtc                                                     17

<210>  6
<211>  17
<212>  DNA
<213>  Artificial

<220>
<223>  Amorce 2

<400>  6
gatttcgttc ctcgacc                                                     17

<210>  7
<211>  23
<212>  DNA

<213>  Artificial

<220>
<223>  Amorce V1


<220>
<221>  misc_feature
<222>  (3)..(3)
<223>  n is a, c, g, or t

<220>
<221>  misc_feature
<222>  (12)..(12)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<400>  7
ggngargayg gnrsnhtnca rgg                                              23


<210>  8
<211>  20
<212>  DNA
<213>  Artificial

<220>
<223>  Amorce V2


<220>
<221>  misc_feature
<222>  (6)..(6)
<223>  n is a, c, g, or t

<220>
<221>  misc_feature
<222>  (9)..(9)
<223>  n is a, c, g, or t

<220>
<221>  misc_feature
<222>  (12)..(12)
<223>  n is a, c, g, or t

<220>
<221>  misc_feature
<222>  (15)..(15)
<223>  n is a, c, g, or t

<400>  8
tgraanccng gnadngtrtt                                                  20


<210>  9
<211>  1079
<212>  DNA
<213>  Enterococcus faecalis / gène ddl

<220>
<221> CDS
<222> (33)..(1079)

<400> 9
aaagacagga aagaaactag gaggacaagc at ttg aag att att ttg ttg tat 53
Leu Lys Ile Ile Leu Leu Tyr
1               5

ggc ggc aga agt gaa gag cac gat gtg tct gtt ttg tct gca tat tcc 101
Gly Gly Arg Ser Glu Glu His Asp Val Ser Val Leu Ser Ala Tyr Ser
        10                  15                  20

gtt tta aat gca atc tat tat aaa tat tat caa gta cag tta gtc ttt 149
Val Leu Asn Ala Ile Tyr Tyr Lys Tyr Tyr Gln Val Gln Leu Val Phe
    25                  30                  35

att agt aaa gac ggt caa tgg gta aaa ggc cct ctt tta tct gaa cga 197
Ile Ser Lys Asp Gly Gln Trp Val Lys Gly Pro Leu Leu Ser Glu Arg
40                  45                  50                  55

cca caa aat aaa gaa gtt tta cat tta act tgg gca caa aca cct gaa 245
Pro Gln Asn Lys Glu Val Leu His Leu Thr Trp Ala Gln Thr Pro Glu
                60                  65                  70

gaa aca ggc gaa ttt tca gga aaa cga atc agt cct tcg gaa att tat 293
Glu Thr Gly Glu Phe Ser Gly Lys Arg Ile Ser Pro Ser Glu Ile Tyr
            75                  80                  85

gaa gaa gaa gcg att gtt ttc cct gtt tta cat ggg cca aat ggt gaa 341
Glu Glu Glu Ala Ile Val Phe Pro Val Leu His Gly Pro Asn Gly Glu
        90                  95                  100

gat gga aca att caa gga ttc atg gaa acc att aat atg cct tat gta 389
Asp Gly Thr Ile Gln Gly Phe Met Glu Thr Ile Asn Met Pro Tyr Val
    105                 110                 115

ggc gcg ggt gtc tta gct agc gtt aac gca atg gac aaa atc atg acg 437
Gly Ala Gly Val Leu Ala Ser Val Asn Ala Met Asp Lys Ile Met Thr
120                 125                 130                 135

aaa tat ctt tta caa act gtt ggc att cca caa gta cca ttc gtg cca 485
Lys Tyr Leu Leu Gln Thr Val Gly Ile Pro Gln Val Pro Phe Val Pro
                140                 145                 150

gtt tta aga agt gac tgg aaa gga aat cca aaa gaa gtc ttt gaa aaa 533
Val Leu Arg Ser Asp Trp Lys Gly Asn Pro Lys Glu Val Phe Glu Lys
            155                 160                 165

tgt gaa ggt tct tta att tat ccg gtc ttt gtt aaa cct gcc aat atg 581
Cys Glu Gly Ser Leu Ile Tyr Pro Val Phe Val Lys Pro Ala Asn Met
        170                 175                 180

ggt tct agt gtc gga att agc aaa gtg gaa aat cgt gaa gaa ttg caa 629
Gly Ser Ser Val Gly Ile Ser Lys Val Glu Asn Arg Glu Glu Leu Gln
    185                 190                 195

gaa gca ttg gaa gaa gct ttc cgt tat gat gcc cga gca att gtt gaa 677
Glu Ala Leu Glu Glu Ala Phe Arg Tyr Asp Ala Arg Ala Ile Val Glu
200                 205                 210                 215

caa ggg atc gaa gca cgt gaa att gaa gta gcc att tta gga aat gaa 725
Gln Gly Ile Glu Ala Arg Glu Ile Glu Val Ala Ile Leu Gly Asn Glu
                220                 225                 230

gat gtc cgt acg act tta cct ggt gaa gtg gtg aaa gat gtc gct ttc 773
Asp Val Arg Thr Thr Leu Pro Gly Glu Val Val Lys Asp Val Ala Phe

            235                 240                 245

tat gat tat gat gca aaa tac atc aat aac acg att gaa atg caa atc 821
Tyr Asp Tyr Asp Ala Lys Tyr Ile Asn Asn Thr Ile Glu Met Gln Ile
        250                 255                 260

cca gcg cat gtt cca gaa gaa gta gct cat caa gcg caa gaa tac gct 869
Pro Ala His Val Pro Glu Glu Val Ala His Gln Ala Gln Glu Tyr Ala
    265                 270                 275

aaa aaa gcg tat att atg tta gat gga agt ggc tta agt cgc tgt gat 917
Lys Lys Ala Tyr Ile Met Leu Asp Gly Ser Gly Leu Ser Arg Cys Asp
280                 285                 290                 295

ttc ttc tta aca agc aaa aac gaa tta ttc ctg aat gaa ttg aac acc 965
Phe Phe Leu Thr Ser Lys Asn Glu Leu Phe Leu Asn Glu Leu Asn Thr
                300                 305                 310

atg cct ggt ttt act gac ttt agt atg tat cct tta ctg tgg gaa aat 1013
Met Pro Gly Phe Thr Asp Phe Ser Met Tyr Pro Leu Leu Trp Glu Asn
            315                 320                 325

atg ggc ttg aaa tac agt gat tta att gag gaa ctg att cag tta gct 1061
Met Gly Leu Lys Tyr Ser Asp Leu Ile Glu Glu Leu Ile Gln Leu Ala
        330                 335                 340

ttg aat cgt ttt aaa taa 1079
Leu Asn Arg Phe Lys
    345

<210> 10
<211> 348
<212> PRT
<213> Enterococcus faecalis / gène ddl

<400> 10

Leu Lys Ile Ile Leu Leu Tyr Gly Gly Arg Ser Glu Glu His Asp Val
1               5                   10                  15

Ser Val Leu Ser Ala Tyr Ser Val Leu Asn Ala Ile Tyr Tyr Lys Tyr
            20                  25                  30

Tyr Gln Val Gln Leu Val Phe Ile Ser Lys Asp Gly Gln Trp Val Lys
        35                  40                  45

Gly Pro Leu Leu Ser Glu Arg Pro Gln Asn Lys Glu Val Leu His Leu
    50                  55                  60

Thr Trp Ala Gln Thr Pro Glu Glu Thr Gly Glu Phe Ser Gly Lys Arg
65                  70                  75                  80

Ile Ser Pro Ser Glu Ile Tyr Glu Glu Glu Ala Ile Val Phe Pro Val
                85                  90                  95

Leu His Gly Pro Asn Gly Glu Asp Gly Thr Ile Gln Gly Phe Met Glu
            100                 105                 110

Thr Ile Asn Met Pro Tyr Val Gly Ala Gly Val Leu Ala Ser Val Asn
        115                 120                 125

Ala Met Asp Lys Ile Met Thr Lys Tyr Leu Leu Gln Thr Val Gly Ile
    130                 135                 140

Pro Gln Val Pro Phe Val Pro Val Leu Arg Ser Asp Trp Lys Gly Asn
145                 150                 155                 160

Pro Lys Glu Val Phe Glu Lys Cys Glu Gly Ser Leu Ile Tyr Pro Val
                165                 170                 175

Phe Val Lys Pro Ala Asn Met Gly Ser Ser Val Gly Ile Ser Lys Val
            180                 185                 190

Glu Asn Arg Glu Glu Leu Gln Glu Ala Leu Glu Glu Ala Phe Arg Tyr
        195                 200                 205

Asp Ala Arg Ala Ile Val Glu Gln Gly Ile Glu Ala Arg Glu Ile Glu
    210                 215                 220

Val Ala Ile Leu Gly Asn Glu Asp Val Arg Thr Thr Leu Pro Gly Glu
225                 230                 235                 240

Val Val Lys Asp Val Ala Phe Tyr Asp Tyr Asp Ala Lys Tyr Ile Asn
                245                 250                 255

Asn Thr Ile Glu Met Gln Ile Pro Ala His Val Pro Glu Glu Val Ala
            260                 265                 270

His Gln Ala Gln Glu Tyr Ala Lys Lys Ala Tyr Ile Met Leu Asp Gly
        275                 280                 285

Ser Gly Leu Ser Arg Cys Asp Phe Phe Leu Thr Ser Lys Asn Glu Leu
    290                 295                 300

Phe Leu Asn Glu Leu Asn Thr Met Pro Gly Phe Thr Asp Phe Ser Met
305                 310                 315                 320

Tyr Pro Leu Leu Trp Glu Asn Met Gly Leu Lys Tyr Ser Asp Leu Ile
                325                 330                 335

Glu Glu Leu Ile Gln Leu Ala Leu Asn Arg Phe Lys
            340                 345

## Description

L'invention concerne les polypeptides associés à l'expression d'une résistance à des antibiotiques de la famille des glycopeptides, cette résistance étant de type inductible par la vancomycine et non inductible par la teicoplanine, notamment chez des bactéries à Gram-positif, en particulier de la famille des cocci à Gram-positif. L'invention vise également une séquence nucléotidique codant pour ces polypeptides. Elle concerne aussi l'utilisation de ces polypeptides et de leur séquence nucléotidique en tant que moyens de détection in vitro d'une résistance à des glycopeptides. Parmi les cocci à Gram-positif, l'invention vise tout particulièrement les entérocoques, les streptocoques et les staphylocoques.

Les glycopeptides, parmi lesquels la vancomycine et la teicoplanine, sont des antibiotiques inhibiteurs de la synthèse de la paroi bactérienne. Ces antibiotiques sont très utilisés pour le traitement des infections sévères dues à des cocci à Gram-positif (entérocoques, streptocoques et staphylocoques), en particulier dans les cas d'allergie et de résistance aux pénicillines.

Jusqu'en 1986, la vancomycine s'est avérée efficace contre la quasi-totalité des souches d'entérocoques.

L'activité des glycopeptides dépend de la formation d'un complexe entre l'antibiotique et les précurseurs du peptidoglycane, plus que de l'interaction directe avec des enzymes du métabolisme de la paroi cellulaire. En particulier on a constaté que les glycopeptides se fixent aux résidus terminaux D-alanyl-D-alanine (D-ala-D-ala) des précurseurs du peptidoglycane.

Plusieurs phénotypes de résistance aux glycopeptides ont été mis en évidence ; on a notamment distingué des souches résistantes à un haut niveau de glycopeptides et les souches résistantes à un bas niveau de concentration.

Par souche résistante à un haut niveau, on entend une souche de bactérie en particulier une souche de cocci a Gram-positif pour laquelle les concentrations minimales inhibitrices (CMI) de vancomycine et de teicoplanine sont respectivement supérieures à 32 et 8 µg/ml. Les CMI de la vancomycine vis à vis des souches résistantes à bas niveau sont comprises entre 8 et 32 µg/ml. Le phénotype VanB est caractérisé par une résistance inductible par la vancomycine, mais non inductible par la teicoplanine. Une fois induite, cette résistance peut exister vis à vis de différents glycopeptides, en particulier vis à vis de la vancomycine et/ou de la teicoplanine, et ce à des niveaux variables.

Les souches d'entérocoques répondant au phénotype VanB (classe B) sont notamment des souches de E.faecalis et E.faecium.

Al-Obeid S. et al (FEMS Microbiology Letters 70 (1990) 101-106) ont ainsi comparé les protéines de résistance à des glycopeptides, inductibles par la vancomycine, chez quatre souches Enterococci, et ont déduit de leur comparaison, l'existence de trois types de protéines, l'un de ces types étant présent chez la souche E.faecium résistante à bas niveau de vancomycine. Selon les auteurs de cette publication, une protéine de poids moléculaire d'environ 39,5 kDa serait induite chez les souches à bas niveau de résistance et cette résistance serait liée à une induction par la vancomycine. Ces souches présenteraient aussi une résistance à la teicoplanine, également induite par la vancomycine.

D'après Al-Obeid et al, cette protéine de 39,5 kDa serait présente sous de multiples formes mais la nature de cette multiplicité n'a pas été étudiée. Selon ces auteurs, il pourrait exister une spécificité de structure en fonction des espèces concernées de bactéries et du niveau de résistance, ce qui demande à être confirmé.

Dans cette publication, Al-Obeid et al ont décrit 11 acides aminés de la séquence N-terminale de cette protéine de 39,5 kDa et ont observé que cette séquence comportait environ 70% d'homologie avec de nombreuses protéines de membrane d'origine procaryote ou eucaryote, ayant des fonctions diverses. Cette comparaison ne permettait pas selon les auteurs d'établir la fonction éventuelle de la protéine. Enfin Al-Obeid et al ont remarqué que d'autres protéines sont induites, à un degré moindre cependant.

L'invention concerne des peptides, polypeptides ou protéines impliqués dans l'expression d'une résistance à des antibiotiques de la famille des glycopeptides et notamment à la vancomycine et/ou à la teicoplanine, ainsi que les séquences nucléotidiques codant pour de tels polypeptides. La résistance dont il est question ci-dessus est de type inductible par la vancomycine et non par la teicoplanine.

Les expressions "impliqué dans l'expression d'une résistance" ou "impliqué dans une résistance" signifient que la protéine de l'invention est nécessaire pour que se manifeste la résistance.

L'invention vise également des sondes nucléotidiques utilisables pour la détection d'une résistance aux glycopeptides, notamment par la réaction de polymérisation en chaîne (PCR), ou par des tests faisant intervenir des anticorps.

L'invention a donc pour objet une protéine VanB **caractérisée en ce qu**'elle comprend la séquence d'acides aminés suivante I, et en ce qu'elle participe à la résistance à des glycopeptides, en particulier à la vancomycine, cette résistance étant de type inductible par la vancomycine et non par la teicoplanine, chez des bactéries à Gram-positif.

M N K I K V A I I F G G C S E E H D V S V K S A I E I A
A N I N T E K F D P H Y I G I T K N G V W K L C K K P C
T E W E A D S L P A I F S P D R K T H G L L V M K E R E
Y E T R R I D V A F P V L H G K C G E D G A I Q G L F E
L S G I P Y V G C D I Q S S A A C M D K S L A Y I L T K
N A G I A V P E F Q M I E K G D K P E A R T L T Y P V F
V K P A R S G S S F G V T K V N S T E E L N A A I E A A
G Q Y D G K I L I E Q A I S G C E V G C A V M G N E D D
L I V G E V D Q I R L S H G I F R I H Q E N E P E K G S
E N A M I I V P A D I P V E E R N R V Q E T A K K V Y R
V L G C R G L A R V D L F L Q E D G G I V L N E V N T L
P G F T S Y S R Y P R M A A A A G I T L P A L I D S L I
T L A I E R

Par l'expression "résistance inductible" on entend la capacité pour une bactérie à Gram-positif déterminée, en particulier pour une souche Enterococcus déterminée, de produire une protéine VanB en présence d'une concentration 0,05 à 1 µl/ml de vancomycine.

La résistance à un ou plusieurs glycopeptides déterminés peut se traduire par la persistance d'une infection due à des germes habituellement sensibles aux glycopeptides, ou peut être détectée au moyen d'un antibiogramme (surtout pour les hauts niveaux de résistance), de la CMI, de l'hybridation avec des sondes (après amplification par exemple par PCR).

Selon un premier mode particulier de réalisation de l'invention, la protéine VanB est **caractérisée en ce qu**'elle est impliquée dans une résistance de type inductible à des glycopeptides et en particulier à la vancomycine, chez des entérocoques et par exemple chez des souches du genre E. faecium ou E. faecalis.

L'invention concerne également une protéine VanB **caractérisée en ce qu**'elle comprend une séquence d'acides aminés modifiée par rapport à la séquence I, par délétion, insertion, ou remplacement d'un ou plusieurs acides aminés, dès lors que la protéine VanB ainsi modifiée est impliquée chez des bactéries à Gram-positif, dans une résistance à des glycopeptides, en particulier à la vancomycine, cette résistance étant de type inductible par la vancomycine, et non inductible par la teicoplanine.

Entre également dans le cadre de l'invention, tout fragment peptidique de la protéine VanB **caractérisé en ce qu**'il correspond à la séquence d'acides aminés I ou à toute partie de cette séquence fonctionnellement associée à la résistance de type inductible à des glycopeptides, en particulier à la vancomycine, chez des bactéries à Gram-positif, par exemple des bactéries de la famille des enterocoques.

Avantageusement des fragments peptidiques de l'invention présentent en outre ou alternativement, des propriétés antigéniques et sont donc reconnus par des anticorps formés contre la protéine VanB.

Un fragment particulier de la séquence I correspond par exemple à la séquence suivante, ou comprend cette séquence :
L F E L S G I P Y V G C D I Q S S A A C M D K S L A Y I L T K N A G I A V P E F Q M I E K G D K P E A R T L T Y P V F V K P A R S G S S F G V T K V N S T E E L N A A I E A A G Q Y D G K I L I E Q A I S G C E V G C A V M G N E D D L I V G E V D Q I R L S H G I F R I H Q E N E P E K G S E N A M I I V P A D I P V E E R N R V Q E T A K K V Y R V L G C R G L A R V D L F L Q E D G G I V L N E V

Selon un autre mode de réalisation de l'invention, ces antigènes sont spécifiques de la protéine VanB et ne sont donc pas reconnus par des anticorps reconnaissant les protéines VanA et VanC telles que décrites dans la demande de brevet EP 91920753.

L'invention concerne en outre une séquence de nucléotides **caractérisée en ce qu**'elle code pour une protéine VanB impliquée chez des bactéries à Gram-positif, dans une résistance à des glycopeptides, en particulier à la vancomycine, cette résistance étant de type inductible par la vancomycine et non inductible par la teicoplanine, ladite protéine VanB comprenant la séquence d'acides aminés I, ou en ce qu'il s'agit d'une séquence d'ADN complémentaire de cette séquence codante, ou d'une séquence d'ARN correspondante.

Par séquence complémentaire, on entend toute séquence d'ADN dont les nucléotides sont complémentaires de ceux de la séquence I, et dont l'orientation est inversée.

Une séquence nucléotidique particulière répondant à cette définition est **caractérisée en ce qu**'elle comprend l'enchaînement de nucléotides II suivant ou un enchaînement nucléotidique modifié par rapport à II, dès lors qu'il code pour une protéine impliquée chez des bactéries à Gram-positif, dans une résistance à des glycopeptides, en particulier à la vancomycine, cette résistance étant de type inductible par la vancomycine et non inductible par la teicoplanine.

GAGCGTGTGCTGCGAGATACCACAGAAAACAATCAGAATTGTCTTAACTTTGAAA
GGAGTTTACAGCATGAATAAAATAAAAGTCGCAATTATCTTCGGCGGTTGCTCGG
AGGAACATGATGTGTCGGTAAAATCCGCAATAGAAATTGCTGCGAACATTAATAC
TGAAAAATTCGATCCGCACTACATCGGAATTACAAAAAACGGCGTATGGAAGCTA
TGCAAGAAGCCATGTACGGAATGGAAGCCGATAGTCTCCCCGCCATATTCTCCC
CGGATAGGAAAACGCATGGTCTGCTTGTCATGAAAGAAAGAGAATACGAAACTCG
GCGTATTGACGTGGCTTTCCCGGTTTTGCATGGCAAATGCGGGGAGGATGGTGCG
ATACAGGGTCTGTTTGAATTGTCTGGTATCCCCTATGTAGGCTGCGATATTCAAA
GCTCCGCAGCTTGCATGGACAAATCACTGGCCTACATTCTTACAAAAAATGCGGG
CATCGCCGTCCCCGAATTTCAAATGATTGAAAAAGGTGACAAACCGGAGGCGAGG
ACGCTTACCTACCCTGTCTTTGTGAAGCCGGCACGGTCAGGTTCGTCCTTTGGCG
TAACCAAAGTAAACAGTACGGAAGAACTAAACGCTGCGATAGAAGCAGCAGGACA
ATATGATGGAAAAATCTTAATTGAGCAAGCGATTTCGGGCTGTGAGGTCGGCTGC
GCGGTCATGGGAAACGAGGATGATTTGATTGTCGGCGAAGTGGATCAAATCCGGT
TGAGCCACGGTATCTTCCGCATCCATCAGGAAAACGAGCCGGAAAAAGGCTCAGA
GAATGCGATGATTATCGTTCCAGCAGACATTCCGGTCGAGGAACGAAATCGGGTG
CAAGAAACGGCAAAGAAAGTATATCGGGTGCTTGGATGCAGAGGGCTTGCTCGTG
TTGATCTTTTTTTGCAGGAGGATGGCGGCATCGTTCTAAACGAGGTCCAATACCC
TGCCCGGTTTTACATCGTACAGCCGCTATCCACGCATGGCGGCTGCCGCAGGAAT
CACGCTTCCCGCACTAATTGACAGCCTGATTACATTGGCGATAGAGAGGTGACCC
GTATGGAAAATGGTTTTTTGTTTTTTAGATGAAATGTTGCA

De manière générale l'invention a également pour objet un fragment nucléotidique **caractérisé en ce qu**'il est capable d'hybrider dans des conditions stringentes avec une séquence telle que définie dans l'enchaînement II ci-dessus.

Les conditions stringentes sont les suivantes :
* température de la réaction 65°C pendant une nuit dans une solution contenant 0,1% de SDS, 0,7% de lait en poudre écrémé, 6xSSC (1xSSC=0,15M NaCl et 0,015M de citrate de sodium à pH=7,0)
* lavages à température ambiante dans 2xSSC-0,1% SDS puis à 65°C dans 0,2SSC-0,1% SDS.

Avantageusement un fragment nucléotidique répondant à la définition précédente aura au moins 15 nucléotides, de préférence au moins 20.

Une séquence nucléotidique particulière comprend à cet égard l'enchaînement suivant:

TCTGTTTGAATTGTCTGGTATCCCCTATGTAGGCTGCGATATTCAAA
GCTCCGCAGCTTGCATGGACAAATCACTGGCCTACATTCTTACAAAAAATGCGGG
CATCGCCGTCCCCGAATTTCAAATGATTGAAAAAGGTGACAAACCGGAGGCGAGG
ACGCTTACCTACCCTGTCTTTGTGAAGCCGGCACGGTCAGGTTCGTCCTTTGGCG
TAACCAAAGTAAACAGTACGGAAGAACTAAACGCTGCGATAGAAGCAGCAGGACA
ATATGATGGAAAAATCTTAATTGAGCAAGCGATTTCGGGCTGTGAGGTCGGCTGC
GCGGTCATGGGAAACGAGGATGATTTGATTGTCGGCGAAGTGGATCAAATCCGGT
TGAGCCACGGTATCTTCCGCATCCATCAGGAAAACGAGCCGGAAAAAGGCTCAGA
GAATGCGATGATTATCGTTCCAGCAGACATTCCGGTCGAGGAACGAAATCGGGTG
CAAGAAACGGCAAAGAAAGTATATCGGGTGCTTGGATGCAGAGGGCTTGCTCGTG
TTGATCTTTTTTTGCAGGAGGATGGCGGCATCGTTCTAAACGAGGTC

Les peptides et polypeptides de l'invention permettent de définir une classe génotypique, caractérisée par la capacité des séquences nucléotidiques codant pour ces peptides, d'hybrider dans des conditions stringentes avec la séquence II constituant une sonde.

Ces fragments peuvent être mis en oeuvre en tant qu'amorces pour la réalisation de réactions d'amplification, ou en tant que sondes.

Des amorces particulièrement intéressantes répondent aux séquences suivantes :
- amorce 1 : 5' ATGGGAAGCCGATAGTC 3' (positions 241-258 des nucléotides de la séquence I)
- amorce 2 : 5' GATTTCGTTCCTCGACC 3' (séquence réverse-complémentaire du fragment des nucléotides 860 à 877 de la séquence I).

Des sondes nucléotidiques selon l'invention peuvent être spécifiques pour détecter chez des bactéries à Gram-positif des séquences codant pour une protéine VanB impliquée dans la résistance à des glycopeptides notamment à la vancomycine et/ou à la teicoplanine, cette résistance étant inductible conformément à la définition précédente, ces sondes pouvant en outre être universelles parmi ces séquences.

Par les sondes spécifiques de vanB, on entend tout oligonucléotide hybridant avec une séquence nucléotidique codant pour une protéine VanB selon l'invention, telle que décrite dans les pages précédentes, et ne présentant pas de réaction d'hybridation croisée ou d'amplification (PCR) avec des séquences présentes chez l'ensemble des souches sensibles.

Un fragment nucléotidique particulier selon l'invention est **caractérisé en ce qu**'il n'hybride pas dans des conditions stringentes avec l'ADN de souches d'entérocoques sensibles à la vancomycine, en particulier avec l'ADN des souches E. faecalis JH2-2 et E. faecium BM4107.

Ces souches de référence ont respectivement été décrites par Jacobs et Hobbs (J. Bacteriol. 117, 1974, 360-372) et Leclercq et al (Antimicrob. Agents Chemother 33, 1989).

Un autre fragment nucléotidique intéressant dans le cadre de l'invention, est spécifique du gène vanB, dans la mesure où il n'hybride pas dans des conditions stringentes avec les gènes vanA et vanC tels que décrits dans la demande PCT 91920753.

Un fragment nucléotidique particulièrement intéressant dans le cadre de l'invention, est le fragment répondant à la séquence II.

Ce fragment est un fragment interne issu du gène impliqué dans la résistance à des souches d'entérocoques. La résistance peut exister à des niveaux variables de concentration en glycopeptides.

L'invention concerne aussi des fragments de nucléotides modifiés par rapport aux précédents, par mutation, addition ou délétion de nucléotides, dès lors que le fragment ainsi modifié soit code pour un fragment de la protéine VanB fonctionnel s'agissant de son association à la résistance à des glycopeptides, en particulier à la vancomycine, dans les conditions décrites ci-dessus, soit hybride avec le gène vanB.

Dans l'hypothèse d'une utilisation des fragments nucléotidiques à titre de sondes, un marquage sera effectué, par les techniques classiques. A titre d'exemple, on utilisera des marqueurs radioactifs ou enzymatiques.

Des fragments de nucléotides selon l'invention peuvent être mis en oeuvre en tant qu'amorces, pour réaliser l'amplification, par exemple par PCR, d'acide nucléique contenu dans un échantillon biologique donné.

L'invention vise par ailleurs une séquence d'ADN recombinante, **caractérisée en ce qu**'elle comprend une séquence de nucléotides ci-dessus décrite, sous le contrôle d'éléments de régulation susceptibles d'intervenir dans le clonage ou l'expression, d'un gène impliqué dans une résistance de type inductible par la vancomycine et non inductible par la teicoplanine, à des antibiotiques de la famille des glycopeptides, notamment la vancomycine, chez un hôte déterminé.

Ce gène impliqué dans la résistance est par exemple le gène vanB qui comprend la séquence de nucléotides II ou toute partie fonctionnelle en terme de résistance inductible, issue d'une séquence hybridant avec la séquence II.

L'invention concerne aussi un vecteur recombinant pour le clonage ou l'expression, **caractérisé en ce qu**'il comprend une séquence nucléotidique ci-dessus décrite en un site non essentiel pour sa réplication, le cas échéant sous le contrôle d'éléments de régulation susceptibles d'intervenir dans l'expression d'une résistance de type inductible par la vancomycine et non inductible par la teicoplanine, à des antibiotiques de la famille des glycopeptides, notamment la vancomycine, chez un hôte déterminé.

Des vecteurs particuliers sont par exemple des plasmides, des phages, des cosmides, des YAC.

Un vecteur préféré est le plasmide pAT201, déposé à la C.N.C.M. le 11 Décembre 1992 sous le numéro I-1277.

Un autre vecteur préféré est le plasmide pAT202 formé à partir du plasmide pUC19Ω contenant un fragment de 3,3 kb contenant le gène vanB de Enterococcus faecalis V583 (HindIII/KpnI).

pAT202 a été introduit dans E. coli JM83 et déposé à la CNCM le 29 Mars 1993, sous le n° I-1291 (identification E. coli BM2973).

Ces vecteurs peuvent être utilisés pour transformer ou transfecter des hôtes cellulaires afin de cloner ou d'exprimer les séquences nucléotidiques de l'invention.

Un hôte cellulaire recombinant selon l'invention est **caractérisé en ce qu**'il est modifié par une séquence nucléotidique ou un vecteur ci-dessus décrits.

De préférence l'hôte cellulaire est modifié par cette séquence dans des conditions permettant l'expression d'une protéine VanB fonctionnelle s'agissant de la résistance inductible à des glycopeptides.

L'invention a aussi pour objet une protéine VanB recombinante telle qu'obtenue à partir d'un hôte cellulaire recombinant selon la définition précédente, la protéine VanB obtenue étant **caractérisée en ce que** son ossature peptidique comprend la séquence d'acides aminés ci-dessus, et en ce qu'elle est impliquée chez des bactéries à Gram-positif, dans une résistance à des glycopeptides, en particulier à la vancomycine, cette résistance étant de type inductible par la vancomycine et non inductible par la teicoplanine.

La protéine VanB selon l'invention permet de préparer des anticorps monoclonaux ou polyclonaux **caractérisés en ce qu**'ils reconnaissent spécifiquement la protéine VanB ou un fragment peptidique décrit ci-dessus.

Ces anticorps peuvent être obtenus selon les méthodes classiques de production d'anticorps. En particulier pour la préparation des anticorps monoclonaux on aura recours à la méthode de Kohler et Milstein selon laquelle on prépare des anticorps monoclonaux par fusion cellulaire entre des cellules de myélome et des cellules spléniques de souris préalablement immunisées avec un polypeptide ou une composition selon l'invention, conformément au procédé classique.

Les anticorps de l'invention sont avantageusement utilisables pour la détection de la présence de protéines caractéristiques d'une résistance aux glycopeptides, en particulier à la vancomycine et à la teicoplanine, cette résistance étant du type inductible par la vancomycine et non inductible par la teicoplanine.

Entre également dans le cadre de l'invention, un kit pour le diagnostic in vitro sur un échantillon biologique, de la présence de souches résistantes à des glycopeptides après induction notamment par la vancomycine et non par la teicoplanine, ces souches appartenant en particulier aux cocci à Gram-positif, **notamment en ce qu**'il s'agit des souches d'entérocoques, par exemple E. faecium, **caractérisé en ce qu**'il comprend :
- des anticorps ci-dessus décrits, le cas échéant marqués,
- un réactif pour la détection d'une réaction immunologique du type anticorps-antigène,
- le cas échéant des réactifs pour effectuer la lyse des cellules de l'échantillon testé,
- le cas échéant une concentration déterminée de vancomycine, pour induire la résistance.

L'invention concerne aussi un kit tel que défini ci-dessus, qui comprend en outre des anticorps dirigés spécifiquement contre la protéine VanA et/ou des anticorps dirigés spécifiquement contre la protéine VanC.

Selon un autre mode de réalisation de l'invention, le kit permet indifféremment la détection d'une résistance correspondant à un phénotype VanA, VanB ou VanC, et comprend des anticorps reconnaissant VanA, VanB et VanC. Ces anticorps peuvent être sélectionnés par leur capacité à reconnaître un épitope commun aux trois protéines. Il peut également s'agir d'un mélange d'anticorps reconnaissant des épitopes différents, propres à chacune des protéines.

Selon une autre mode de réalisation de l'invention, un kit pour le diagnostic in vitro de la présence de souches résistantes à bas niveau, à des glycopeptides, en particulier résistantes à la vancomycine, est **caractérisé en ce qu**'il comprend :
- une sonde nucléotidique capable d'hybrider dans des conditions stringentes avec une séquence nucléotidique du gène vanB, et le cas échéant,
- des nucléoside-triphosphates dATP, dCTP, dTTP, dGTP,
- un agent de polymérisation d'ADN.

Un autre kit de détection comprend en outre une sonde de nucléotides capable d'hybrider spécifiquement avec le gène vanA et une sonde capable d'hybrider spécifiquement avec le gène vanC.

Ce kit peut être avantageusement utilisé pour la détection d'une résistance chez des cocci à Gram-positif notamment chez des entérocoques, par exemple chez E. faecium.

L'invention vise aussi un kit pour la détection in vitro d'une résistance à des glycoprotéines, notamment à la vancomycine, cette résistance correspondant à l'un des phénotypes VanA, VanB ou VanC, le kit comprenant
- une sonde nucléotidique hybridant avec les gènes vanA, vanB et vanC,
- des nucléoside-triphosphates dATP, dCTP, dTTP et dGTP,
- un agent de polymérisation d'ADN.

L'invention vise aussi un procédé de détection in vitro de la présence de souches résistantes à des glycopeptides, en particulier à la vancomycine et/ou à la teicoplanine, ces souches appartenant en particulier à la famille des cocci à Gram-positif, notamment en ce qu'il s'agit de souches d'entérocoques, par exemple E. faecium ou E.faecalis, **caractérisé en ce qu**'il comprend :
a) la mise en contact d'un échantillon biologique susceptible de contenir les souches résistantes, avec une amorce constituée par un fragment nucléotidique selon l'invention, tel que décrit ci-dessus, capable d'hybrider avec une séquence nucléotidique recherchée, impliquée dans l'expression de la résistance, cette séquence étant utilisée comme matrice, en présence des 4 différents nucléosides triphosphates, et d'un agent de polymérisation, dans des conditions d'hybridation telles que pour chaque séquence nucléotidique ayant hybridé avec une amorce, un produit d'élongation de chaque amorce complémentaire de la matrice est synthétisé,
b) la séparation de la matrice et du produit d'élongation obtenu, ce dernier pouvant alors également se comporter comme une matrice,
c) la répétition de l'étape a) de façon à obtenir une quantité détectable des séquences nucléotidiques recherchées,
d) la détection du produit d'amplification des séquences nucléotidiques.

La sonde utilisée peut donc être spécifique de la séquence nucléotidique II ou d'une séquence hybridant dans des conditions stringentes avec la séquence II. Dans ces conditions, le procédé selon l'invention permet la détection d'une résistance à des glycopeptides, cette résistance étant inductible par la vancomycine et non inductible par la teicoplanine.

Selon un mode de réalisation particulier de l'invention, ce procédé comprend également la mise en contact de l'échantillon biologique avec un fragment nucléotidique spécifique du gène vanA et/ou un fragment nucléotidique spécifique du gène vanC. Dans ce cas le procédé selon l'invention permet avantageusement la détection de différents phénotypes de résistance.

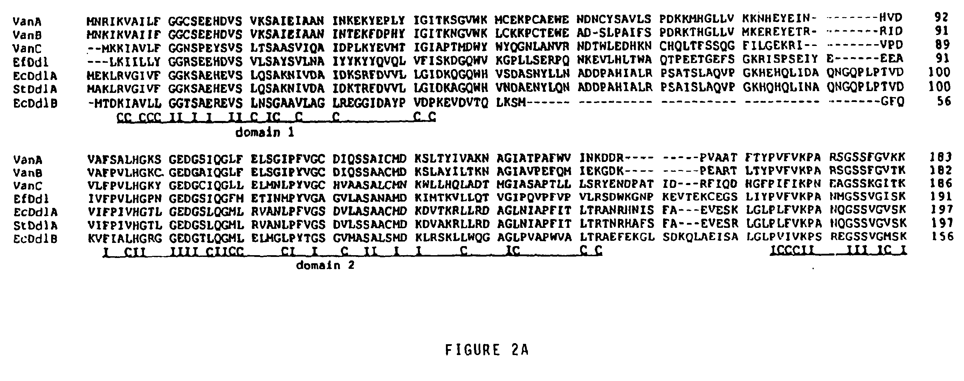

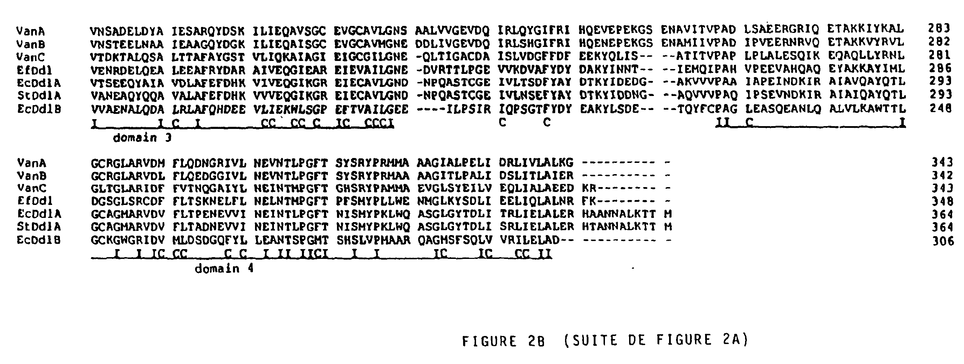

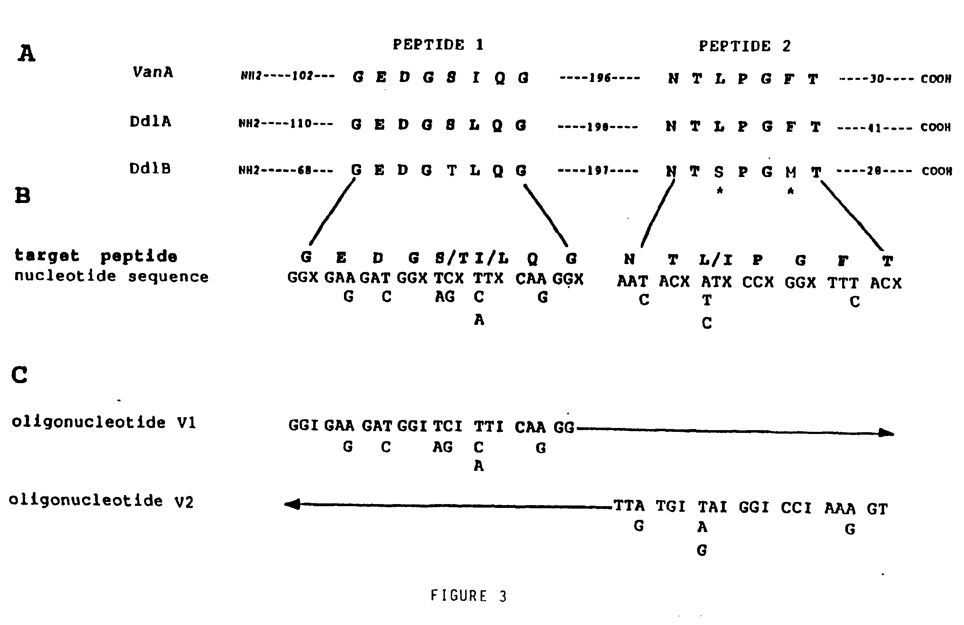

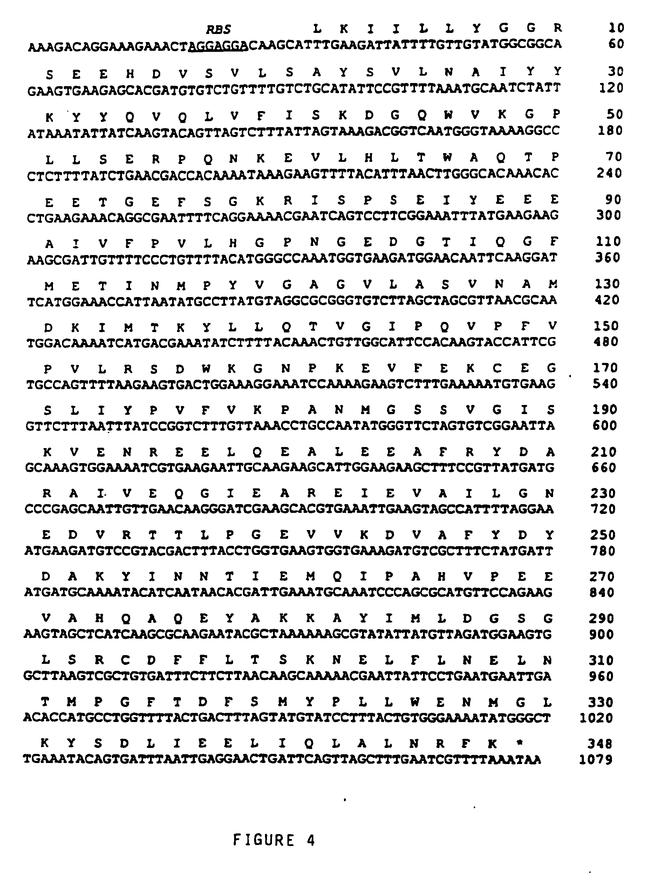

Selon un autre mode de réalisation, on détectera indifféremment une résistance correspondant à un phénotype VanA, VanB ou VanC, en utilisant une sonde commune aux gènes vanA, vanB ou vanC. Une telle sonde peut être réalisée à partir des séquences polypeptidiques alignées de la figure 2.

D'autres caractéristiques et avantages de l'invention apparaissent dans les exemples et les figures suivants :

### FIGURES

Figure 1 :
   Séquence de nucléotides et d'acides aminés correspondant au gène vanB. La séquence de nucléotides des deux brins a été déterminée à partir de l'insérat contenu dans pUC18, par la méthode didéoxy de terminaison de chaîne (Sanger et al, 1977, Proc. Natl. Acad. Sci. USA, 74:5463-5467) en utilisant l'ADN polymérase T7.
   La séquence soulignée RBS représente la séquence Shine-Dalgarno de liaison au ribosome.
Figure 2 :
   Alignement de la séquence d'acides aminés déduite de la protéine VanB et des régions correspondantes de VanA, VanC, DdLA et DdLB de E.coli (Dutka-Malen et al, 1992 Gene, 112:53-58), Ddl de En.faecalis V583 et DdlA de S.typhimurium (Daub et al - Biochemistry 27, 1988, 3701-3708). Les acides aminés identiques (I) et les substitutions conservatives (C) dans les 7 séquences ont été indiqués sous l'alignement. Pour que la classification en tant que substitution soit conservée, les acides aminés ont été regroupés comme suit : RK, LFPMVI, STQNC, AGW, H, ED et Y.
   Les domaines 1, 2, 3 et 4 correspondent à des régions de forte homologie.
Figure 3 :
   Oligonucléotides V1 et V2 utilisés pour amplifier l'ADN du gène vanB.
Figure 4 :
   Séquence de nucléotides du gène ddl de En.faecalis V583 et séquence d'acides aminés correspondante. Le plasmide pAT203 a été construit en sous-clonant l'ADN d'un bactériophage λ recombinant partiellement digéré avec Sau3AI (Pharmacia), dans pUC19 digéré avec BamHI (Pharmacia). L'insérat de 15 kb de pAT203 comprend le gène ddl. La séquence de nucléotides de 1079 pb consécutives de pAT203 a été déterminée sur les deux brins par la méthode dideoxy de terminaison de chaine. La première paire de bases de la séquence est définie à la position 1. La séquence de liaison au ribosome RBS est à la position 19 en amont du codon de départ TTG. Le codon stop TTA est indiqué. La séquence d'acides aminés déduite de la protéine Ddl est présentée.

### APPROCHE EXPERIMENTALE

Les antibiotiques de la famille des glycopeptides, tels que la vancomycine (Vm) et la teicoplanine (Te) se fixent aux résidus D-Ala C-terminaux des précurseurs du peptidoglycane, bloquant ainsi leur incorporation dans la paroi cellulaire bactérienne (Reynolds, P.E. 1989 Eur. J. Clin. Microbiol. Infect. Dis. 8:943-950). Les résidus D-Ala sont incorporés dans les précurseurs au niveau de la paroi cellulaire, sous forme de dipeptides synthétisés par des ligases D-Ala:D-Ala (DDL) (Walsh, C.T. 1989 J. Biol. Chem. 264:2393-2396). La ligase VanA synthétise le dipeptide D-Ala-D-lac qui se substitue à D-Ala-D-Ala conduisant à la synthèse de précurseurs qui lient la vancomycine avec une affinité réduite (Bugg et al, Biochemistry 30:10408-10415 (1991), Handwerger et al, J. Bacteriol. 174:5982-5984 (1992), Messer et Reynolds, FEMS Microbiol. Letters 94:195-200 (1992)).

La résistance aux glycopeptides chez les entérocoques est hétérogène (Dutka-Malen et al, 1990 Antimicrobiol Agents Chemother 34:1875-1879).

Les protéines de résistance VanA et VanC (voir demande de brevet EP 91920753.0 du 29 octobre 1991) montrent une homologie de 30 à 37% (les détails sont donnés dans le tableau III) en acides aminés avec les D-Ala:D-Ala ligases (Ala=Alanine) de E.coli (Dutka-Malen et al, 1992 Gene 112:53-58). Les gènes de structure pour les protéines VanA et VanC n'hybrident pas avec l'ADN des souches de phénotype VanB (Dutka-Malen et al, 1990, Leclercq et al, Antimicrob. Agents Chemother 36:2005-2008 (1992)).

Les inventeurs sont parvenus à identifier la séquence nucléotidique impliquée dans les propriétés de résistance à la vancomycine de souches d'entérocoques ayant le phénotype VanB et résistant, après induction avec la vancomycine.

Souches Bactériennes : 39 isolats de E.faecium (28 souches) et E.faecalis (11 souches) résistantes à de faibles ou de fortes concentrations de vancomycine et sensibles à la teicoplanine ont été étudiées (tableau II). Parmi ces souches, 24 isolats y compris E.faecalis V583 (Sahm D. et al, Antimicrob. Agents Chemother. 1989, 33:1588-91) et E.faecium D366 (Gutmann L. et al, Antimicrob. Agents Chemother 1992, 36:77-80) étaient résistantes à de faibles concentrations de vancomycine sur la base d'un test de sensibilité sur disque. Ces souches appartiennent au phénotype de classe B. 15 isolats résistant à de fortes concentrations de vancomycine (CIM ≥ 128 µg/ml) y compris E.faecalis souche V583-2 (Zarlenga LJ. et al, Antimicrob. Agents Chemother. 1992, 36:902-5), qui est un mutant spontané de V553, ainsi que UMH-1 (Schwalbe R. et al, Abstract A-117, in Abstracts of the 91st General Meeting of the American Society for Microbiology. Dallas, TX: American Society for Microbiology, 1991) ont aussi été étudiés. Les souches-contrôle étaient des souches d'enterocoque bien caractérisées appartenant aux phénotypes A et C et hybridant avec les sondes VanA et VanC respectivement. Il s'agit en particulier de 6 isolats cliniques de E.faecium hautement résistants à la vancomycine et à la teicoplanine, incluant BM4147. Les souches de E.qallinarum incluant BM4174 appartenant à la classe C ont également été utilisées comme contrôle. Font également partie les contrôles de souches de E.casseliflavus, y compris la souche ATCC 25788, qui sont des isolats intrinsèquement résistants à de faibles niveaux de vancomycine et sensibles à la teicoplanine (Leclercq R. et al, Antimicrob. Agents Chemother. 1992, 36:2005-8).

Les souches suivantes ont également été étudiées : Erysipelothrix rhusiopathiae A 124 (Institut Pasteur Collection), Lactobacillus brevis ATCC 14869, Lactobacillus casei ATCC 393, Lactobacillus confusus ATCC 10881, Lactobacillus fermentum ATCC 9338, Lactobacillus plantarum ATCC 8014, Lactobacillus reuteri ATCC 23272, Lactobacillus rhamnosus ATCC 7469, Lactobacillus salivarius ATCC 11741, Pediococcus acidilacti ATCC 8042, Pediococcus pentosaceus ATCC 33316, et Leuconostoc mesenteroides CIP 16407. Les enterocoques suivants sensibles à des antibiotiques de la famille des glycopeptides sont utilisés comme contrôles négatifs: E.durans ATCC 19432, E.faecium ATCC 19434, BM4107 (Leclercq R. et al, Antimicrob. Agents Chemother. 1992, 36:2005-8), et MT10R (GutmannL et al, Antimicrob. Agents Chemother, 1992, 36:77-80), souche sensible à la vancomycine derivée de D366 ; E.faecalis ATCC 29212, ATCC 33186,JH2-2 (Leclercq R. et al, Antimicrob. Agents Chemother. 1992, 36:2005-8), et V583-C1, souche sensible à la vancomycine dérivée de V583, (Tableau II), et un isolat clinique de E.faecium et E.faecalis. Des caractéristiques de souches de référence sont dépeintes au tableau I. E.faecium BM4107 et E.faecalis JH2-2, à la fois résistantes à la rifampine et à l'acide fusidique (Leclercq R. et al, Antimicrob. Agents Chemother. 1992, 36:2005-8), étaient utilisés comme souches réceptrices pour des expériences de conjugaison.

### Identification des entérocoques

Les entérocoques ont été identifiés par la méthode de Facklam et Collins, J. Clin. Microbiol. 1989, 27:731-4). L'identification des espèces était fondée sur les tests de réduction du tellurite de potassium et de production d'acides à partir de carbohydrates sur des bandes de streptocoques API 20 (bioMérieux, Marcy l'Etoile, France). Le test de mobilité à 30°C et de fermentation des carbohydrates ont été utilisés pour distinguer E.gallinarum et E.casseliflavus de E.faecium et E.faecalis. Les souches de E.casseliflavus ont été distinguées des souches de E.gallinarum sur la base de la production d'un pigment jaune sur de l'agar.

### Milieu

Un milieu coeur-cerveau et de l'agar (Difco Laboratoires, Détroit, MI) ont été utilisés. Des tests de sensibilité ont été réalisés sur de l'agar Mueller-Hinton (Diagnostics Pasteur, Marne La Coquette, France). Toutes les incubations ont été réalisées à 37°C.

### Détermination de la sensibilité in vitro aux antibiotiques.

Le test de diffusion sur disque avec des disques contenant 30 µg de vancomycine ou 30 µg de teicoplanine (Diagnostics Pasteur) a été utilisé pour le criblage initial. La méthode de Steers et al avec 10⁴ CFU par tâche a été utilisée pour déterminer la CMI des antibiotiques (Steers E. et al, Antibiot. Chemother. (Basel) 1959, 9:307-11).

### Transfert du caractère de résistance d'un antibiotique

La conjugaison sur filtres a été réalisée selon la technique décrite par Dutka-Malen S. et al, Antimicrob. Agents Chemother. 1990, 34:1875-9. Les concentrations en antibiotiques pour la sélection des transconjugués étaient les suivantes : rifampine : 20 µg/ml ; acide fusidique : 10µg/ml et vancomycine : 4 et 8 µg/ml.

### Enzymes et réactifs

La lisozyme a été obtenue chez Sigma Chemical Co. (St Louis, MO). La RNase A (pancréas de bovin) et la protéinase K ont été obtenues chez Calbiochem. Co. (San Diego, CA). L'[α-³²P]dCTP et le sel de triethylammonium (activité spécifique 3000 CI/mmol) ont été obtenus de Radiochemical Center, Amersham, Grande-Bretagne. La teicoplanine provient de Gruppo Lepetit (Milan, Italie) et la vancomycine provient de Eli Lilly & Co (Indianapolis, IN).

Les oligonucléotides V1 et V2 décrits dans la demande de brevet EP 91920753.0, ont permis l'amplification par la technique de PCR, de fragments internes aux gènes codant pour les protéines VanA, VanC et D-Ala:D-Ala ligases (Dutka-Malen et al, 1992 Gene 112:53-58).

L'amplification du gène vanB a été réalisée avec les oligonucléotides V1 et V2 et l'ADN (20 ng) de Enterococcus faecalis V583 (Sahm et al, 1989 Antimicrob. Agents Chemother 33:1588-1591).

Pour réaliser cette amplification, la technique décrite dans la publication de Dutka-Malen et al., 1992 a été utilisée. Les fragments obtenus ont été séparés sur gel d'agarose (1%) dans un tampon TAE, qui a permis de révéler une bande unique d'environ 600 pb, qui a été extraite du gel en utilisant un kit de purification de l'ADN (GeneClean, Bio101 Inc., la Jolla, CA). En utilisant un kit permettant l'obtention d'extrémités franches au niveau de l'ADN (Amersham, Amersham, Grande-Bretagne), les fragments ont été traités avec l'ADN T4 polymérase et ligaturés au niveau du site SmaI d'un plasmide pUC18 digéré et déphosphorilé (Norrander et al, 1983, Gene 26:101-106).

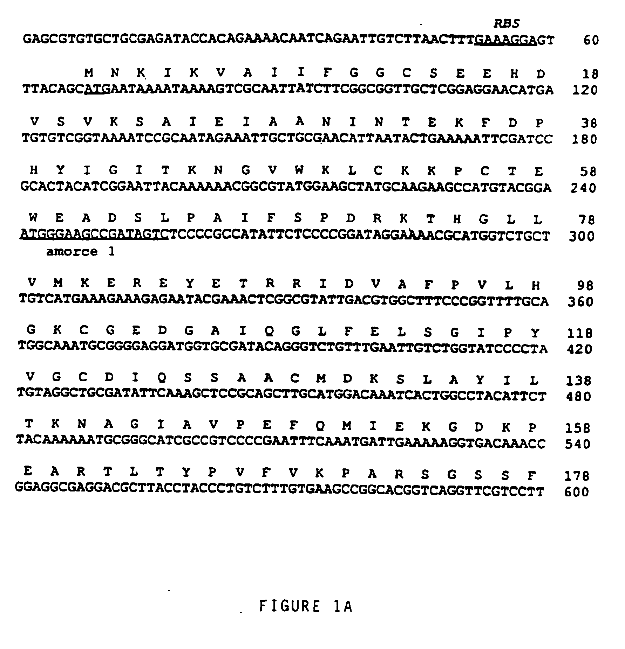

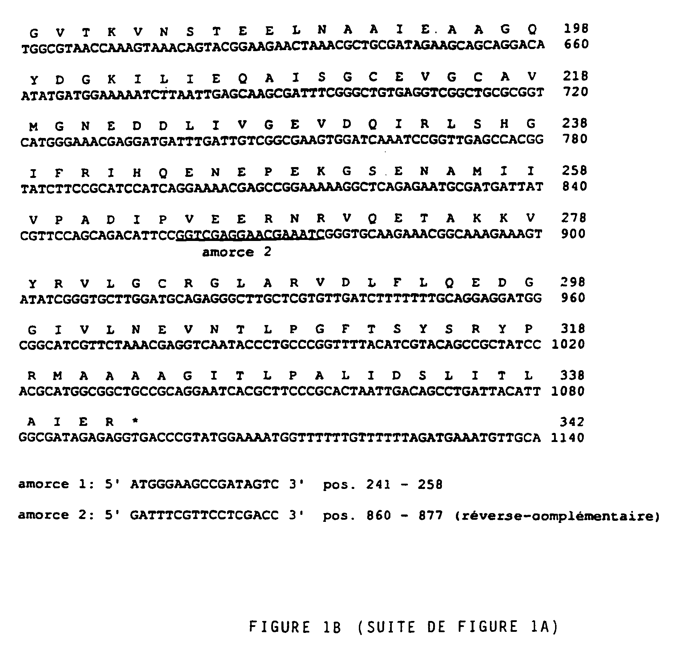

La séquence de 632 pb (sonde vanB) correspondant à l'insert du plasmide recombinant (figure 1) a été déterminée par la méthode didéoxy de terminaison de chaîne (Sanger et al, 1977, Proc. Natl. Acad. Sci. USA, 74:5463-5467) en utilisant l'ADN T7 polymérase (Pharmacia, Uppsala, Suède) et le [α-³⁵S]dATP (Amersham, Radiochemical Center, Amersham, Grande-Bretagne).

Etant donné que l'amplification avec l'ADN Taq polymérase peut conduire à des incorporations erronées de nucléotides, la séquence a été confirmée comme suit : un oligonucléotide complémentaire des positions 513 à 530 de la séquence nucléotidique de la figure 1 a été synthétisé par la méthode au phosphoramidite (unité de Chimie Organique, Institut Pasteur, France) et utilisé avec l'amorce V1 pour réaliser une amplification par PCR d'un fragment de vanB. Le produit PCR a été séquencé directement (Mabilat et al, 1990, Plasmid 23:27-34) ou après le clonage dans un vecteur pUC18, afin de révéler l'identité des nucléotides avec le fragment cloné obtenu avec V1 et V2.

Une hybridation de type southern a été réalisée selon la méthode de Johnson et al, Gene Anal. Téchnol. 1:3-8 (1984). l'ADN total des souches d'entérocoque (Tableau 1) a été préparé selon la technique décrite par Le Bouguénec et al, 1990, J. Bacteriol. 172:727-734, digéré avec les enzymes HindIII et KpnI (United States, Biochemical Corporation, Cleveland, Ohio) et résolu sur des gels d'agarose 1% . L'ADN a été transféré sur des membranes de nylon (Nytran, Schleicher & Schuell, Dassel, Allemagne) avec un appareil de transfert sous vide (Trans. Vac. TE80, Hoefer Scientific Instruments, San Francisco, CA). La sonde a été obtenue en marquant le fragment PCR cloné avec un kit de translation de césure (Bethesda Research Laboratories Life Technologies Inc., Gaithersburg, MD) et (α-³²P)dCTP (Amersham, Radiochemical Center, Amersham, Grande-Bretagne). L'hybridation a été réalisée dans des conditions stringentes à 68°C (Johnson et al, 1984, Gene Anal. Technol. 1:3-8). Les membranes ont été lavées à 65°C dans 0,1% SDS-2XSSC.

La sonde vanA consistait en un fragment PstI de 265 pb interne au gène vanA (Dukta Malen S. et al, Mol. Gen. Genet. 1990, 224:364-72). La sonde vanC consistait en un fragment EcoRI-HincII de 690 pb, interne au gène vanC (Leclercq R. et al, Antimicrob. Agents Chemother 1992, 36:2005-8. Dukta Malen S. et al Gene, 1992, 112:53-8). La sonde vanB correspond à la séquence II.

La séquence en acides aminés déduite de l'insérat contenu dans le plasmide pUC18 a été comparée avec différentes séquences de protéines [figure 2] : le tableau 5 résume les pourcentages d'identité en acides aminés comparés deux à deux pour les séquences des protéines VanB, VanA, VanC, ElDdl, DdlA et DdlB. Dans les conditions d'hybridation en southern, le fragment cloné hybridait avec le fragment de 3,3 kb HindIII-KpnI de E. faecalis V583. La sonde n'hybride pas avec l'ADN d'un dérivé de V583 sensible à la vancomycine ou à l'ADN des souches de E. faecalis et E. faecium sensibles à la vancomycine utilisées comme référence. Le fragment d'ADN cloné obtenu par PCR correspond à un fragment interne du gène impliqué dans la résistance. Ce gène code pour une enzyme apparentée aux D-Ala:D-Ala ligases, appelée VanB, qui pourrait être impliquée dans la synthèse d'un produit se substituant à D-Ala-D-Ala.

Ces tests ont permis de mettre en évidence un seul groupe de gènes apparentés à vanB, et responsable d'un bas et d'un haut niveau de résistance à la vancomycine chez les entérocoques (tableaux 1 et 2).

Aucune hybridation n'a été observée entre la sonde VanB et l'ADN de souches sensibles à la vancomycine sans induction ou portant les gènes vanA ou vanC ou intrinsèquement résistantes.

La séquence complète du gène vanB a été clonée en mettant en oeuvre les étapes suivantes :
Le plasmide pAT202 a été obtenu en sous-clonant dans pUC19 un fragment HindIII-KpnI de 3,3 kb du bactériophage recombinant λ contenant le gène vanB. Le clonage a été réalisé avec des endonucléases de restriction (Boehringer, Mannheim, Allemagne et Pharmacia LKB Biotechnology Inc. Uppsala, Suède), la T4 DNA ligase (Boehringer) et la phosphatase alkaline (Pharmacia) conformément aux recommendations du fabricant. La séquence nucléotidique de 1090 pb consécutives de pAT202 a été déterminée pour les deux brins par la méthode dideoxy de terminaison de chaine (Sanger et al, 1977) en utilisant la T7 DNA polymérase modifiée (Amersham Radiochemical Center, Amersham, Grande-Bretagne) et des oligonucléotides complémentaires de la séquence, synthétisés par la méthode au méthoxy phosphoramidite (Institut Pasteur, Paris, France). Les produits des réactions ont été résolus par électrophorèse sur gel de polyacrylamide 6% dénaturant. La première paire de bases de la séquence présentée correspond à la position 1 (figure 1). Le site potentiel de liaison au ribosome (RBS) (Moran et al, Mol. Gen. Genet. 186 (1982) 339-346) en amont du codon ATG d'initiation à la position 46 est souligné. Le codon stop (TGA) est indiqué par une astérisque. La séquence d'acides aminés est alignée avec le premier nucléotide de chaque codon.
Le transfert du caractère de résistance à la vancomycine (chez 6 isolats d'entérocoques parmi 17) par conjugaison sur filtre, a été observé chez des souches E.faecium et E.faecalis résistantes à de faibles ou à de fortes concentrations d'antibiotiques.
Parmi les autres fragments d'environ 600 bp amplifiés à partir des oligonucléotides V1 et V2, un insérat hybridait avec l'ADN de souches Vm^{R} ou Vm^{S} de En.faecalis mais pas avec l'ADN de souches de 18 autres espèces. Ce gène code pour une D-Ala:D-Ala ligase dans En.faecalis. Puisqu'aucun autre gène de ligase dans En.faecalis n'a été détecté, ce gène a été appelé ddl.
Le clonaqe et le séquençage du gène ddl inséré dans le vecteur pUC19 (Norrander et al, Gene 26, 1983, 101-106) ont permis de constater que la teneur en bases G et C de ddl (37,5%) et du chromosome de En.faecalis (37-39%) étaient très semblables.
Différentes observations suggèrent que le gène vanB aurait une origine exogène : (i) le gène peut être transféré par conjugaison. (ii) Les séquences de nucléotides apparentées à van B n'ont pas été détectées dans l'ADN de souches Vm^{S} de En.faecalis et En.faecium et les représentants de 16 autres espèces d'Enterococcus (tableau III). (iii) La teneur en bases GC du gène vanB diffère de façon marquée de celle du chromosome de En.faecalis. (iv) Le niveau faible de similitude entre Ddl de En.faecalis et VanB (34% d'identité) indique que les gènes correspondants n'ont pas pour origine une duplication récente.

### Précurseurs de peptidoglycanes chez En.faecalis Vm^{R} et Vm^{S}

L'incubation de En.faecalis V583 avant l'induction de souches En.faecalis JH2-2 Vm^{R} ou Vm^{S} (Jacob et Hobbs - J. Bacteriol. 117, 1974, 360-372) avec la ramoplanine (9 µg/ml) inhibiteur de la paroi cellulaire a conduit à l'accumulation du précurseur de la paroi cellulaire UDP-N-acetylmuramyl-L-Ala-D-Glu-L-Lys-D-Ala-D-Ala (UDP-Mur-NAc-pentapeptide) qui est utilisé dans le cycle normal de la synthèse du peptidoglycane.

Après l'induction de la résistance, En.faecalis V583 a accumulé trois intermédiaires de la paroi cellulaire lorsque la souche était incubée avec la ramoplanine (tableau IV). Ces intermédiaires ont été identifiés comme étant des UDP-MurNAc-pentapeptides, UDP-MurNAc-tetrapeptide dépourvu de la partie C-terminale D-Ala de l'UDP-MurNAc-pentapeptide ; et de façon prédominante, UDP-MurNAc-tetrapeptide-D-lactate dans lequel la partie C-terminale D-Ala de l'UDP-MurNAc-pentapeptide est remplacée par le D-lactate. La présence de UDP-MurNAc-tetrapeptide-D-lactate suggère que les souches de phénotypes VanA et VanB ont le même mécanisme de base de résistance aux glycopeptides ; c'est à dire qu'ils synthétisent du D-lactate qui peut être lié à VanB (ou VanA) par le D-Ala pour synthétiser du D-Ala-D-lactate qui est ensuite incorporé dans le précurseur de peptidoglycane.

Les précurseurs de la paroi ont été purifiés par chromatographie d'échange d'ions et désalage par filtration sur gel. L'identification a été fondée sur une spectroscopie de masse (positive ion electrospray mass spectroscopy), un spectre UV (pour l'uracil) analyse automatisée des acides aminés après hydrolyse pendant 4h et 24h (pour l'acide muramique et les taux en acides aminés) et l'analyse par réactions enzymatiques spécifiques du résidu terminal obtenu par réaction avec le D,D-carboxypeptidase de Actinomadura R39 (Messer et Reynolds, FEMS Microbiol Letter 94, 1992, 195-200).

La quantité totale de précurseurs accumulée dans chaque culture était semblable approximativement. 65 µmol/g de poids sec dans une période d'incubation correspondant à 0,6 de la durée moyenne de synthèse.

**TABLEAU I: Souches bactériennes**

| Souche | CMI (µg/ml) | | Hybridation avec la sonde vanB | | | Référence |
|---|---|---|---|---|---|---|
| | Vm | Te | | | | |
| | | | vanA | vanB | vanC | |
| E. faecalis | | | | | | |
| V583 | 64 | 0,5 | - | + | - | Sahm et al (1989) |
| V583-C1 | 2 | 0,5 | - | - | - | D.F. Sahm |
| V583-2 | 1024 | 1,0 | - | + | - | Zarlenga LJ (1992) |
| UMH-1 | 1024 | 1,0 | - | + | - | Schwalbe et al (1991) |
| ATCC 29212 | 2 | 0,5 | - | - | - | |
| | | | | | | |
| E.faecium | | | | | | |
| D366 | 32 | 0,5 | - | + | - | Gutmann et al (1992) |
| MT10R | 2 | 0,25 | - | - | - | Gutmann et al (1992) |
| BM4147 | 1024 | 512 | + | - | - | Dutka-Malen et al (1990) |
| ATCC 19434 | 1 | 1 | - | - | - | |
| | | | | | | |
| E. gallinarum | | | | | | |
| BM4174 | 8 | 1 | - | - | + | Dutka-Malen et al (1992) |
| | | | | | | |
| E.casseliflavus | 8 | 1 | - | - | - | |

**TABLEAU II:**

| **Classes de phénotypes et génotypes parmi les coccins à Gram-positif, résistantes à la vancomycine** | | | | |
|---|---|---|---|---|
| CLASSE DE PHENOTYPE | CLASSE DE (A) GENOTYPE | ESPECE | CMI (µg/ml) | |
| | | | Vancomycine | Teicoplanine |
| Susceptible | Susceptible | Enterococcus spp.(10) | 0.5-2 | 0.25 |
| A | A | E.faecium(6) | 256->1.000 | 64->1.000 |
| B | B | E.faecium(28) | 4-256 | 0.5-1 |
| B | B | E.faecalis(11) | 4-1024 | 0.5-1 |
| C | C | E.gallinarum(3) | 8 | 1 |
| C | NC | E.casseliflavus(2) | 4-8 | 0.5-1 |
| NC | NC | Lactobacillus spp.(8) | >1.000 | >1.000 |
| NC | NC | Leuconostoc. sp.(1) | >1.000 | >1.000 |
| NC | NC | Pediococcus spp.(2) | >1.000 | >1.000 |
| NC | NC | E.rhusiopathiae(1) | >1.000 | >1.000 |

| | | | | |
|---|---|---|---|---|
| ^{(a)}A: hybridation avec la sonde vanA ; B: hybridation avec la sonde vanB ; C: hybridation avec la sonde vanC ; NC : non classé | | | | |

**TABLEAU III Résultats des expériences d'hybridation**

| | Resistance | Nbre de souches testées | Hybridation avec sonde | |
|---|---|---|---|---|
| Espèces | phenotype | | *vanB* | *ddl (En. faecalis)* |
| *En. faecalis* | Vm^{R}, Te^{S} | 11 | + | + |
| | Vm^{S}, Te^{S} | 5 | - | + |
| *En. faecium* | Vm^{R}, Te^{R} | 6 | - | - |
| | Vm^{R}, Te^{S} | 28 | + | - |
| | Vm^{S}, Te^{S} | 4 | - | - |
| *En. gallinarum* | Vm^{R}, Te^{S} | 3 | - | - |
| *En. casseliflavus* | Vm^{R}, Te^{S} | 2 | - | - |
| *En*. spp. (15 espèces^{a}) | Vm^{S}, Te^{S} | 15 | - | - |

| | | | | |
|---|---|---|---|---|
| ^{a} Type de souches de En.avium, En. cecorum, *En. columbae, En. dispar, En. durans, En. flavescens, En. hirae, En. malodoratus, En. mundtii, En. pseudoavium, En. raffinosus, En. saccharolyticus, En. seriolicida, En. solitarius* et *En. sulfureus*. | | | | |

**TABLEAU IV**

| Précurseurs de peptidoglycane dans des souches de En. Faecalis Vm^{S} ou Vm^{R} | | | |
|---|---|---|---|
| Précurseur de Peptidoglycane | Quantité de précurseur (%) de En.faecalis | | |
| | JH2-2 | V583 non-induite | V583 induite |
| UDP-MurNAc-L-Ala-D-Glu- | 100 | 100 | 14 |
| L-Lys-D-Ala-D-Ala | | | |
| UDP-MurNAc-L-Ala-D-Glu- | 0 | 0 | 7 |
| L-Lys-D-Ala | | | |
| UDP-MurNAc-L-Ala-D-Glu- | 0 | 0 | 79 |
| L-Lys-D-Ala-D-lactate | | | |

**TABLEAU V**

| Identité de séquence entre les séquences d'acides aminés de Van b, Ddl de En.faecalis V583 et des D-Ala:D-Ala ligases^{a} | | | | | | |
|---|---|---|---|---|---|---|
| Sequence comparée^{b} | Pourcentage d'identité par rapport à : | | | | | |
| | VanB | VanC | EfDdl | EcDdlA | StDdlA | EcDdlB |
| VanA | 76 | 38 | 32 | 38 | 37 | 30 |
| VanB | | 38 | 34 | 38 | 38 | 32 |
| VanC | | | 34 | 34 | 34 | 36 |
| EfDdl | | | | 40 | 40 | 34 |
| EcDdlA | | | | | 90 | 35 |
| StDdlA | | | | | | 35 |

| | | | | | | |
|---|---|---|---|---|---|---|
| ^{a} Identité des paires de séquences dérivée de l'alignement de la figure 2 ^{b} Ec, E.coli; Ef, En.faecalis; St, S.typhimurium | | | | | | |

## Revendications

1. Procédé de criblage de molécules d'antibiotiques actifs pour le traitement des infections dues à des cocci à Gram-positif, en particulier des entérocoques, **caractérisé en ce que** l'on détecte l'activité de ces molécules, sur des bactéries ayant un phénotype de résistance à la vancomycine, de type VanB.

2. Procédé de criblage selon la revendication 1, dans lequel les bactéries sont **caractérisées par** une résistance inductible par la vancomycine et non inductible par la téicoplanine.

3. Procédé de criblage selon la revendication 1 ou 2, dans lequel les bactéries sont des cocci à gram positifs.

4. Procédé de criblage selon la revendication 3, dans lequel les cocci à gram positifs sont des souches de *E. faecalis* ou de *E. faecium*.

5. Procédé de criblage selon l'une quelconque des revendications 1 à 4, dans lequel les bactéries sont des bactéries recombinantes comprenant une séquence nucléotidique codant pour une protéine VanB impliquée chez des bactéries à Gram-positif, dans une résistance à la vancomycine, cette résistance étant de type inductible par la vancomycine et non inductible par la teicoplanine, ladite protéine VanB comprenant dans son ossature peptidique la séquence d'acides aminés suivante :

M N K I K V A I I F G G C S E E H D V S V K S A I E I A
A N I N T E K F D P H Y I G I T K N G V W K L C K K P C
T E W E A D S L P A I F S P D R K T H G L L V M K E R E
Y E T R R I D V A F P V L H G K C G E D G A I Q G L F E
L S G I P Y V G C D I Q S S A A C M D K S L A Y I L T K
N A G I A V P E F Q M I E K G D K P E A R T L T Y P V F
V K P A R S G S S F G V T K V N S T E E L N A A I E A A
G Q Y D G K I L I E Q A I S G C E V G C A V M G N E D D
L I V G E V D Q I R L S H G I F R I H Q E N E P E K G S

E N A M I I V P A D I P V E E R N R V Q E T A K K V Y R
V L G C R G L A R V D L F L Q E D G G I V L N E V N T L
P G F T S Y S R Y P R M A A A A G I T L P A L I D S L I
T L A I E R

6. Procédé de criblage selon la revendication 5, dans lequel ladite séquence nucléotidique codant pour une protéine VanB comprend l'enchaînement de nucléotides ci-dessous ou un enchaînement nucléotidique modifié par mutation, addition ou délétion de nucléotides par rapport à l'enchaînement de nucléotides ci-dessous, dès lors qu'il code pour une protéine impliquée chez des bactéries à Gram-positif, dans une résistance à la vancomycine, cette résistance étant de type inductible par la vancomycine et non inductible par la teicoplanine :

GAGCGTGTGCTGCGAGATACCACAGAAAACAATCAGAATTGTCTTAACTTTGAAA
GGAGTTTACAGCATGAATAAAATAAAAGTCGCAATTATCTTCGGCGGTTGCTCGG
AGGAACATGATGTGTCGGTAAAATCCGCAATAGAAATTGCTGCGAACATTAATAC
TGAAAAATTCGATCCGCACTACATCGGAATTACAAAAAACGGCGTATGGAAGCTA
TGCAAGAAGCCATGTACGGAATGGGAAGCCGATAGTCTCCCCGCCATATTCTCCC
CGGATAGGAAAACGCATGGTCTGCTTGTCATGAAAGAAAGAGAATACGAAACTCG
GCGTATTGACGTGGCTTTCCCGGTTTTGCATGGCAAATGCGGGGAGGATGGTGCG
ATACAGGGTCTGTTTGAATTGTCTGGTATCCCCTATGTAGGCTGCGATATTCAAA
GCTCCGCAGCTTGCATGGACAAATCACTGGCCTACATTCTTACAAAAAATGCGGG
CATCGCCGTCCCCGAATTTCAAATGATTGAAAAAGGTGACAAACCGGAGGCGAGG
ACGCTTACCTACCCTGTCTTTGTGAAGCCGGCACGGTCAGGTTCGTCCTTTGGCG
TAACCAAAGTAAACAGTACGGAAGAACTAAACGCTGCGATAGAAGCAGCAGGACA
ATATGATGGAAAAATCTTAATTGAGCAAGCGATTTCGGGCTGTGAGGTCGGCTGC
GCGGTCATGGGAAACGAGGATGATTTGATTGTCGGCGAAGTGGATCAAATCCGGT
TGAGCCACGGTATCTTCCGCATCCATCAGGAAAACGAGCCGGAAAAAGGCTCAGA
GAATGCGATGATTATCGTTCCAGCAGACATTCCGGTCGAGGAACGAAATCGGGTG
CAAGAAACGGCAAAGAAAGTATATCGGGTGCTTGGATGCAGAGGGCTTGCTCGTG
TTGATCTTTTTTTGCAGGAGGATGGCGGCATCGTTCTAAACGAGGTCCAATACCC
TGCCCGGTTTTACATCGTACAGCCGCTATCCACGCATGGCGGCTGCCGCAGGAAT
CACGCTTCCCGCACTAATTGACAGCCTGATTACATTGGCGATAGAGAGGTGACCC
GTATGGAAAATGGTTTTTTGTTTTTTAGATGAAATGTTGCA

7. Procédé de criblage selon la revendication 5, dans lequel ladite séquence nucléotidique codant pour une protéine VanB comprend un fragment nucléotidique, **caractérisé en ce qu'**il est capable d'hybrider dans des conditions stringentes avec une séquence nucléotidique définie aux revendications 5 ou 6 ou **en ce qu'**il est complémentaire au fragment nucléotidique hybridant, ledit fragment codant un fragment de la protéine VanB, suffisant pour conférer la résistance à la vancomycine, cette résistance étant de type inductible par la vancomycine et non inductible par la teicoplanine, la protéine vanB étant définie :
a) **en ce qu'**elle comprend la séquence d'acides aminés suivante, et **en ce qu'**elle est impliquée chez des bactéries à Gram-positif, dans une résistance à la vancomycine, cette résistance étant de type inductible par la vancomycine et non inductible par la teicoplanine. 
M N K I K V A I I F G G C S E E H D V S V K S A I E I A
A N I N T E K F D P H Y I G I T K N G V W K L C K K P C
T E W E A D S L P A I F S P D R K T H G L L V M K E R E
Y E T R R I D V A F P V L H G K C G E D G A I Q G L F E
L S G I P Y V G C D I Q S S A A C M D K S L A Y I L T K
N A G I A V P E F Q M I E K G D K P E A R T L T Y P V F
V K P A R S G S S F G V T K V N S T E E L N A A I E A A
G Q Y D G K I L I E Q A I S G C E V G C A V M G N E D D
L I V G E V D Q I R L S H G I F R I H Q E N E P E K G S
E N A M I I V P A D I P V E E R N R V Q E T A K K V Y R
V L G C R G L A R V D L F L Q E D G G I V L N E V N T L
P G F T S Y S R Y P R M A A A A G I T L P A L I D S L I
T L A I E R ;
 ou
b) **en ce qu'**elle comprend une séquence d'acides aminés modifiée par rapport à la séquence telle que définie à l'option a) par délétion ou insertion ou remplacement d'un ou plusieurs acides aminés, dès lors que la protéine VanB ainsi modifiée est nécessaire à l'établissement d'une résistance à la vancomycine chez des bactéries gram-positif, cette résistance étant de type inductible par la vancomycine et non inductible par la teicoplanine.

8. Procédé de criblage selon la revendication 7, **caractérisé en ce que** ledit fragment n'hybride pas dans des conditions stringentes avec l'ADN de souches d'entérocoques sensibles à la vancomycine, en particulier avec l'ADN des souches *E*. *faecalis* JH2-2, *E. faecium* BM4107.

9. Procédé de criblage selon la revendication 8, **caractérisé en ce que** ledit fragment consiste en l'enchaînement nucléotidique illustré dans la revendication 6.

10. Procédé de criblage selon l'une quelconque des revendications 1 à 4, dans lequel les bactéries sont des bactéries recombinantes comprenant une séquence d'ADN recombinante **caractérisée en ce qu'**elle comprend une séquence de nucléotides telle que définie dans l'une quelconque des revendications 5 à 9, sous le contrôle d'éléments de régulation susceptibles d'intervenir dans l'expression d'une résistance à la vancomycine, cette résistance étant inductible par la vancomycine et non inductible par la teicoplanine.

11. Procédé de criblage selon l'une quelconque des revendications 1 à 4, dans lequel les bactéries sont des bactéries recombinantes comprenant un vecteur recombinant pour le clonage ou l'expression dans un hôte déterminé, **caractérisé en ce qu'**il comprend une séquence nucléotidique telle que définie dans l'une quelconque des revendications 5 à 9, en un site non essentiel pour sa réplication, sous le contrôle d'éléments de régulation susceptibles d'intervenir dans l'expression d'une résistance à la vancomycine, cette résistance étant inductible par la vancomycine et non inductible par la teicoplanine.

12. Procédé de criblage selon la revendication 11, dans lequel ledit vecteur est le plasmide pAT201 déposé à la CNCM sous le numéro I-1277 ou le plasmide pAT202 déposé à la C.N.C.M. sous le numéro I-1291.
